(19) Europäisches Patentamt
European Patent Office
Office européen des brevets

(11) Publication number: **0 035 575**
**B1**

(12) # EUROPEAN PATENT SPECIFICATION

(45) Date of publication of patent specification: **08.08.84**

(51) Int. Cl.³: **A 61 K 35/16,**
B 01 J 20/26, G 01 N 33/50

(21) Application number: **80901710.6**

(22) Date of filing: **11.09.80**

(86) International application number:
**PCT/JP80/00206**

(87) International publication number:
**WO 81/00673 19.03.81 Gazette 81/7**

(54) **BLOOD SERUM-SEPARATING AGENT.**

(30) Priority: **11.09.79 JP 116388/79**
**26.05.80 JP 69913/80**

(43) Date of publication of application:
**16.09.81 Bulletin 81/37**

(45) Publication of the grant of the patent:
**08.08.84 Bulletin 84/32**

(84) Designated Contracting States:
**DE NL SE**

(56) References cited:
EP - A - 0 035 575
FR - A - 2 290 665
US - A - 3 972 812
US - A - 4 101 422
US - A - 4 131 549
US - A - 4 153 739

(73) Proprietor: **TERUMO CORPORATION**
**44-1, 2-chome, Hatagaya Shibuya-Ku**
**Tokyo 151 (JP)**

(72) Inventor: **ASADA, Yoshimitsu**
**25-2, Honmachi 5-chome**
**Shibuya-ku Tokyo 151 (JP)**
Inventor: **WATANABE, Teruko**
**27-13, Himonya 5-chome**
**Meguro-ku Tokyo 152 (JP)**
Inventor: **ICHIKAWA, Toshiji**
**9-7, Someji 2 chome**
**Chofu-shi, Tokyo (JP)**

(74) Representative: **Klöpsch, Gerald, Dr.-Ing.**
**An Gross St. Martin 6**
**D-5000 Köln 1 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 035 575 B1**

Courier Press, Leamington Spa, England.

## Description

The present invention relates to a serum-separating agent used for separating serum from other components in a blood.

This serum-separating agent is used to fractionate a specific component from other components using the specific gravity difference between the specific component and other components of a blood to be fractionated, thus being interposed between these components. For this purpose, it is desired that the serum-separating agent have a specific gravity between those of said components, be flowable during centrifugal separation, and not flowable and stable after the operation of centrifugal separation is completed.

A known conventional serum-separating agent of this kind is a gel-like material consisting of silicone oil, silica and a gelling agent. But a liquid separating agent of such a composition has problems. For example, because components which are compatible are mechanically mixed to obtain a thixotropic gel by means of a gelling agent which accelerates the formation of hydrogen bonds between the silica particles (specific gravity adjusting agent), coagulation develops due to this growing hydrogen bond becoming stronger as time elapses, resulting in phase separation and poor fluidity during centrifugal separation. Thus, addition of a surfactant for avoiding phase separation has been proposed, but this presented another problem of hemolysis by the large amount of surfactant.

Further, serum-separating agents of the above composition have drawbacks such as the tendency to change their nature by cross-linking and other chemical changes upon $\gamma$-ray sterilization after encapsulation of a suitable quantity in a blood collecting tube. This degrades the serum-separating function, and delays blood coagulation and clot deposition by evaporation of low molecular components of the gel-like material rendering the inner surface of the tube hydrophobic. Furthermore, these separating agents are fairly expensive.

Another known serum-separating agent is a gel-like material of polyester base.

For example, US—A—4 101 422 claims copolyesters which are derived from mixed dibasic acids and a branched claim diol that permit their use in small amounts in blood separation tubes to provide an effective barrier between the light and heavy phases of blood after centrifuging. These copolyesters form a tight seal against the inner surface of the blood separation assembly. This material is not necessarily satisfactory in that it renders the inner surface of the tube water-repellent resulting in delay of blood coagulation and clot deposition. Furthermore, serum-separating agents of this type smell unpleasant.

The object of the present invention is to provide a novel serum-separating agent which is free from above problems of the prior art. Other object of the present invention is to provide a serum-separating agent which has an excellent stability over long periods of time, can be subjected to $\gamma$-ray sterilization, show virtually no water-repellency, is free of unpleasant odors and can be manufactured at comparatively low cost, and as a result it is possible to eliminate hemolysis and to prevent delay in blood coagulation and clot deposition on the inner surface of a blood-sampling tube, even if blood is preliminarily introduced in the tube.

According to the present invention, there is provided a thixotropic serum separating agent for fractionating serum portion from the coagulated portion of blood, which is characterized by consisting of, as the main component, an $\alpha$-olefin-dialkylmaleate copolymer having a viscosity between 100 and 800 $N \cdot s/m^2$ (at 25°C) and a specific gravity of 1.035—1.055, (28°C) and as the minor component viscosity- and specific gravity-adjusting agents mixed therewith.

In the present invention, typical example of the $\alpha$-olefin-dialkyl maleate copolymer has the general formula:

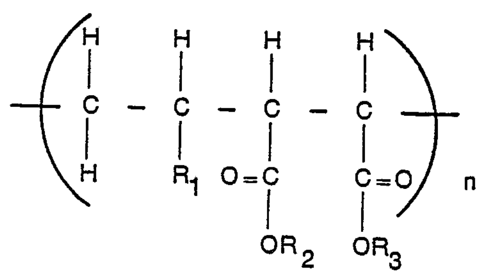

where $R_1$ stands for an alkyl group having 2 to 58 carbon atoms which in the copolymer molecule may either be the same group or a combination of different groups as desired; $R_2$ and $R_3$ are selected from methyl, ethyl, butyl, and 2-ethylhexyl groups; n is an integer which allows the viscosity of said copolymer to be in the range of 100 to 800 more preferably 400 to 800 $N \cdot s/m^2$ (at 25°C) when using said copolymer as the main component, or corresponds to a carbon atom number in the range of 30 to 60 when using said copolymer as a wax.

This α-olefin-dialkylmaleate copolymer is light yellow in color, transparent, odorless, inert to blood, free of blood absorption, elution and so on, and stable for long periods of time. It allows the inner surface of the blood collecting tube to remain clean since it does not produce any water-repellent material. It does not substantially change its chemical or physical nature upon sterilisation with gamma-rays or the like.

When the liquid separation agent of the present invention is used for blood serum separation, the specific gravity of this α-olefin-dialkyl maleate copolymer is selected between 1.00 and 1.038 (28°C), preferably between 1.027 and 1.035 (28°C).

According to the present invention, aliphatic amine derivatives of smectite clay that are used as viscosity and specific gravity adjusting agents, may be aliphatic primary amine, aliphatic secondary amine or aliphatic tertiary amine or aliphatic quaternary amine derivatives of smectite clay. These amine derivatives are already known. Among these derivatives, aliphatic quaternary amine derivatives of smectite clay are most desirable, examples of which are aliphatic amine derivatives of smectite clay having $C_8$—$C_{24}$. Such smectite clays are known under the registered trademarks Benton® 34, Benton® 38, Benton® 27, and Benton® 128 (quaternary ammonium salts of smectite clay, products of NL Industry Co.).

The inorganic fine powder used as the viscosity and specific gravity adjusting agent in the present invention may be suitably selected from calcined silica, precipitated silica and so on, and may be added in view of the specific gravity and viscosity of the main component in such an amount that makes the whole composition into the gel-like and prescribed specific gravity.

The structure-forming agent used in the present invention is added for making and maintaining the gel state of the liquid separation agent, and can be used when it is considered to be difficult to make the whole composition into gel-like without the inclusion of such an additive. For example, dimethylpolysiloxane-polyoxyalkylene copolymer (e.g., trade names SH-3771, SH-190, and SH-192 of Toray Silicone Co., Ltd.) or Carbitol (e.g., ethyldiglycol) may be used. The amount of the structure-forming agent may be decided by taking the kinds of main component and viscosity- and specific gravity-adjusting agent into consideration, so that it is suffice for gelation and miscible with other components.

In the present invention, in addition to the α-olefin-dialkyl maleate copolymer, the viscosity and specific gravity adjusting agent and the structure-forming agent, a nonionic surfactant (e.g., polyoxy-ethylene-hydrogenated caster oil monolaurate or polyoxy-ethylene-hydrogenated caster oil tri-isostearate) may be added as required. The addition of a little amount (for example, 0.47—2.7 weight %) of the surfactant will be effective in preventing the phase separation for a long period of time and since the surfactant is nonionic, there is no possibility of hemolysis. The employment of the surfactant is especially useful where only silica is employed as a viscosity- and specific gravity-adjusting agent.

BRIEF EXPLANATION OF DRAWINGS

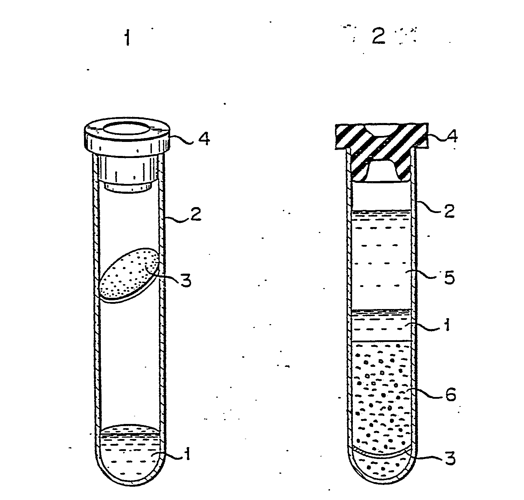

Fig. 1 is a perspective view illustrating the use of the liquid separating agent of the present invention as a blood serum separator encapsulated in a blood collection tube. Fig. 2 is a sectional view of the blood collection tube shown in Fig. 1 after centrifugal separation.

Compositions of the liquid separating agent of the present invention for serum separation are shown in the following tables 1 and 2. In these tables, the α-olefin-dialkyl maleate copolymer (A) is an n-α-olefin-dimethyl maleate copolymer having average molecular weight 3,000—4,000; specific gravity 1.027—1.035 (at 25°C); viscosity 400—700 N·s/m² (25°C) and consists of a combination of α-olefin components with carbon atom numbers 12 and 14, respectively; the copolymer (B) is an n-α-olefin-dimethyl maleate copolymer having average molecular weight 2.000—3.000; specific gravity 1.005 (28°C); viscosity 100—150 N·s/m² (28°C) and consists of a combination of α-olefine components with carbon atom numbers 6 and 8, respectively; the copolymer (C) is a wax made of an n-α-olefin-diamethyl maleate copolymer of α-olefin components with an average carbon atom number between 30 and 60; the copolymer (D) is an n-α-olefin-dimethyl maleate copolymer having average molecular weight 3.600—4.000 specific gravity about 0.995 (28°C), viscosity about 100 N·s/m² (28°C) and consists of a combination of α-olefin components with carbon atom numbers 16 and 18 respectively; and the copolymer (E) shown in Table 2 is an n-α-olefin-dimethyl maleate copolymer having average molecular weight 3.000—4.000; specific gravity 1.027—1.035 (at 25°C); viscosity 400—700 N·s/m² (25°C) and consists of a combination of α-olefin components with carbon atom numbers 12 and 14, respectively (sold under a trade name PAR-24, MITSUBISHI KASEI Industries Ltd.).

Other examples of α-olefin-dialkyl maleate copolymer include, in addition to above-mentioned examples, n-α-olefin-dibutyl maleate copolymer and n-α-olefin-di-2-ethylhexyl maleate copolymer.

TABLE 1

(examples of serum separation agent compositions) (parts by weight)

| Composition Number | 1 | 2 | 3 | 4 | 5 | 6 | 7 | 8 |
|---|---|---|---|---|---|---|---|---|
| α-olefin-dialkylmaleate copolymer | | | | | | | | |
| (A) | 100 | 100 | 100 | 100 | 50 | – | | |
| (B) | – | – | – | – | 50 | 100 | | |
| (C) | – | – | 1.0 | 3.0 | – | – | | |
| (D) | – | – | – | – | – | – | 100 | 100 |
| Aerosil® R–972* | | – | 2.0 | 2.0 | 1.0 | | 1.5 | |
| Aerosil® 200** | 1.0 | 1.0 | – | – | – | – | | |
| Benton® 38*** | – | 1.0 | 1.0 | – | 4.0 | 8.0 | 10.0 | |
| Benton® 34*** | – | – | – | – | 1.0 | – | – | 12.0 |
| Benton® 27*** | 3.0 | – | – | – | – | – | – | – |
| Benton® 128*** | – | – | 2.0 | – | – | – | – | – |
| Specific gravity (28°C) | 1.047 | 1.039 | 1.052 | 1.040 | 1.042 | 1.036 | 1.041 | 1.041 |
| Viscosity (100 N·s/m²) | 53 | 62 | 58 | 43 | 57 | 75 | 64 | 69 |
| Hemolysis | none | none | none | none | none | none | none | none |
| Adhesion of clot | none | none | none | none | none | none | none | none |

* hydrophobic silica fine powder of average particle size 16 mμ, apparent specific gravity about 60 g/l, (product of NIPPON AEROSIL CO., LTD.)

** hydrophilic silica fine powder of average particle size 12 mμ, apparent specific gravity about 60 g/l, (product of NIPPON AEROSIL CO., LTD.)

*** quaternary ammonium salts of smectite clay, (products of NL Industry CO., U.S.A.)

TABLE 2

(examples of serum separation agent compositions) (parts by weight)

| Composition Number | 7 | 8 | 9 | 10 | 11 | 12 |
|---|---|---|---|---|---|---|
| α-olefin-dialkylmaleate copolymer | | | | | | |
| (E) | 100 | 100 | 100 | 100 | 50 | – |
| (B) | – | – | – | – | 50 | 100 |
| (C) | – | – | 5.0 | 5.0 | – | – |
| Aerosil® R–972* | 6 | – | 7.0 | 5.0 | 7.0 | 7 |
| Aerosil® 200** | – | 6 | – | – | – | – |
| Surfactant*** | 0.5 | 1.0 | 1.0 | – | 1.0 | 3.0 |
| Structure-forming agent**** | 0.18 | 0.04 | – | 0.2 | 0.5 | 0.6 |
| Specific gravity (28°C) | 1.042 | 1.042 | 1.045 | 1.040 | 1.045 | 1.042 |
| Viscosity (100 $N \cdot s/m^2$/25°C) | 54 | 80 | 73 | 68 | 52 | 51 |
| Hemolysis | none | none | none | none | none | none |
| Adhesion of clot | none | none | none | none | none | none |

* see Table 1.

** see Table 1.

*** Polyoxyethylene-hydrogenated caster oil monolaurate, (product of NIHON EMULSION CO., LTD.)

**** SH–3771, (product of Toray Silicone Co., Ltd.), dimethylpolysiloxane-polyoxyalkylene copolymer with specific gravity 1.060 – 1.080 (20°C) and viscosity 2,6 – 2,8 $N \cdot s/m^2$.

Next, the preparation method of the liquid separating agent of the present invention will be described.

First, the manufacturing method of the α-olefin-dialkyl maleate copolymer is low polymerization of ethylene to obtain an n-α-olefin. This is then separated into fractions of carbon atom numbers, for example, of 4, of 6, of 8 and 10, of 12 and 14, of 16 and 18, and of 30 to 60 by fractional distillation. In accordance with the specific gravity of the liquid to be fractionated, these fractions may be used either singly or in combination, and a fraction with carbon atom numbers of 12 and 14, or of 6 and 8 is preferable for use in serum separation from the standpoints of viscosity and specific gravity. The selected fraction is subjected to copolymerization with a maleic acid diester in the conventional manner to obtain the desired product.

Using this α-olefin-dialkyl maleate copolymer with a viscosity between 100 and 800 $N \cdot s/m^2$, preferably between 400 and 800 $N \cdot s/m^2$ (25°C) as the base a viscosity and specific gravity adjusting agent such as an aliphatic amine derivative of smectite clay, fine silica powder, a structure-forming agent, nonionic surfactant and a wax consisting of an α-olefin-maleic-diester copolymer (for example, above n-α-olefin having 60—80 carbon atoms) are added as required. The mixture is kneaded using either a roll mill, a grinding mill or a planetary mixer.

The liquid separating agent thus prepared should preferably has a viscosity, for use in serum separation for example, between 250 and 800 $N \cdot s/m^2$ (25°C) and a specific gravity between 1.035 and 1.055 (28°C). All of the components in the previous tables are thixotropic, showing flowability upon application of a centrifugal force or the like and staying in the normal condition as a stable uniform gel otherwise.

As apparent from above Tables 1 and 2, even if blood is preliminarily kept in a blood sampling tube, the inner surface of the tube does not become water-repellent, thus preventing the adhesion of clots or delay of blood coagulation. These advantages can be attributed to the employment of α-olefin-maleate diester copolymer which does not generate low molecular volatile materials. Accordingly, when serum-separating agent is employed in a blood-sampling tube, it is possible to form a stable seal at the interference between serum layer and clot layer by an ordinary centrifuging operation of 10 minutes at 700—1000 G. As a result, inclusion of fibrin can be easily avoided in a fractionating operation of serum by decantation. Further, since the serum-separating agent is stably positioned between serum layer and clot layer after the centrifuging operation, there is little chance of the clot layer mixing into the serum layer during the transportation of blood from a hospital to an examination center and the like.

Following is an example of using a serum-separating agent preliminarily introduced in a blood sampling-tube.

As shown in Fig. 1, about 1.7 ml of each liquid separating agent composition 1 above (numbers 1 to 12) was put in the bottom of a 10 ml blood collecting tube 2. A polyester unwoven cloth 3 coated with 1—5 mg of diatomaceous earth (e.g., Caper Flattery Sand, trade name WG-200, for Kyoritsu Ceramic Materials Co., Ltd.) or micro-glass powder, was then placed at a slant in each blood collecting tube. Each tube was then stoppered with a butyl rubber plug 4, and the tubes were placed under reduced pressure. Then a blood sample was placed in each blood collecting tube and allowed to stand for 7 to 8 minutes. As a result, upon the introduction of the blood the diatomaceous earth is dispersed in the blood and together with the unwoven cloth 3 it accelerates blood coagulation. Adequate coagulation was thus attained within this short time. Each blood collecting tube was placed in a centrifuge for 10 minutes at 700—1,000 G, and the liquid separating agent compositions were stably distributed between the serum and the clot layers. This state is shown in Fig. 2. Because the liquid separating agent is thixotropic and has a specific gravity between that of the blood serum 5 and that of the blood clots 6, it stays between the blood serum 5 and blood clot 6, forming a gel that separates these two layers. Since the diatomaceous earth and the unwoven cloth 3 have higher specific gravities, they were not included in the layer of blood serum 5. Thus, blood serum 5 obtained was of high purity with no entrainment of fibrin. This blood serum 5 was readily collected from the blood collecting tube by decantation or by suction through a fine nozzle.

In the above example, the case wherein the serum-separating agent is preliminarily introduced into a sampling tube is explained. However, this invention is not limited to the above example. For example, the serum-separating agent may be provided together with its container at an upper portion of a blood sampling tube after the sampling of blood and taking-off of rubber cap. In this case the serum-separating agent is introduced from the container into the blood-sampling tube by a centrifuging operation and forms a seal at the interface between serum portion and clot portion.

When being used as a serum separator, the liquid separating agent does not form a water-repellent film in the blood collecting tube by releasing water-repellent substances and consequently does not cause delay in blood coagulation. Because the blood collecting tube is made of glass, which accelerates coagulation when contacting blood at the surface, so that it is necessary to keep the inner surface clean. Accordingly, compared with conventional liquid separating agents which have the drawback of forming water-repellent film, the time needed for collecting blood serum is shortened. This is even more effective in combination with the use of the diatomaceous earth and unwoven cloth. The time saving can amount to as much as 30 minutes.

When encapsulation in a blood collecting tube is performed, sterilization is desirable. In clinical tests, no chemical or physical changes that cause adverse effects were found after applying gamma-ray sterilization a dose of 2.5· megarad.

α-olefin-dialkyl maleate copolymers having alkyl groups containing 4 to 60 carbon atoms in the α-olefin portion and maleate ester containing an alkyl group selected from methyl, ethyl, butyl and 2-ethylhexyl groups, and having a viscosity of 100 to 800 $N\cdot s/m^2$ (25°C) are very stable for a long period of time. Accordingly, when these copolymers are employed as main components and mixed with viscosity- and specific gravity-adjusting agents for preparing serum separating agents, the obtained mixtures are easily gelled, and adjusted to an intermediate specific gravity between those of serum and clot, and do not substantially separate into or become less fluid.

Further, as shown in the above composition (Table 1), when $C_8$—$C_{24}$ aliphatic amine derivative of smectite or fine powderous inorganic materials such as hydrophobic silica powder, as a viscosity-specific gravity-adjusting agent there will be obtained a serum separating agent which is free from phase separation, degradation of fluidity and has stable thixotropic characteristics. If a serum separating agent includes $C_8$—$C_{24}$ aliphatic amine derivative of smectite clay, the state of dispersion in the agent can easily be checked by means of a microscope, making quality control easier, because the smectite clay particles are not light-transmitting.

Because the aliphatic amine derivatives of smectite clay such as quaternary ammonium salt derivatives of smectite have a thickening effect as shown in Table 1, it is possible to obtain a stable thixotropic serum separating agent without employing a structure-forming agent.

Among the examples shown in the above Table 1 and 2, α-olefin-dialkyl maleate copolymer (A), (B), (C) and (E) are all n-α-olefin-dimethyl maleate copolymer and, when employed as a main component in a serum separating agent, make it possible to easily obtain a serum separating agent of nearly an intermediate specific gravity between those of serum and clot and having a suitable degree of viscosity, requiring smaller amount of a viscosity- and specific gravity-adjusting agent being added. Furthermore, these compositions will be substantially free from changes with time, phase separation and lowering of fluidity.

The above mentioned wax is especially useful for preventing a serum separating agent from separating into phases.

## Claims

1. A serum separating agent having thixotropic characteristics for fractionating a serum component from a coagulated portion by means of centrifugation, characterised by comprising, as the main component, an α-olefin-dialkyl maleate copolymer having a viscosity of 100 to 800 $N\cdot s/m^2$ (25°C) and as minor component an agent to control viscosity and specific gravity mixed therewith for adjusting the specific gravity of the whole composition to 1.035 to 1.055 (28°C).

2. The serum-separating agent according to claim 1 wherein said α-olefin-dialkyl maleate copolymer has the general formula:

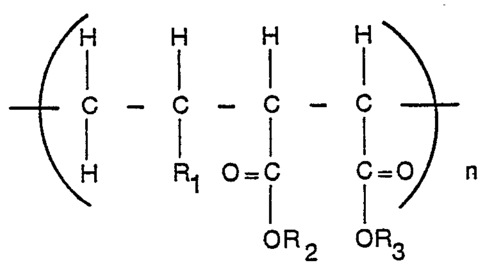

where $R_1$ is an alkyl group having 2 to 58 carbon atoms which, in the copolymer molecule, may either be the same group or a combination of different groups as desired; $R_2$ and $R_3$ are selected from methyl, ethyl, butyl and 2-ethylhexyl groups, and n is an integer.

3. The serum separating agent according to claim 1 or 2 wherein said agents to control viscosity and specific gravity are selected from aliphatic amine derivatives of smectite clay with the carbon atom numbers between 8 and 24 and inorganic fine powders.

4. The serum-separating agent according to claim 3 wherein said aliphatic amine derivatives of smectite clay are quaternary ammonium salts of smectite clay.

5. The serum-separating agent according to claim 3 wherein said inorganic fine powders are calcined silica or precipitated silica.

6. The serum-separating agent according to claim 2 wherein $R_2$ and $R_3$ in said general formula are methyl groups.

7. The serum-separating agent according to claim 6 wherein $R_1$ in said general formula is a combination of alkyl groups having 10 and 12 carbon atoms.

8. The serum-separating agent according to claim 6 wherein $R_1$ in said general formula is a combination of alkyl groups having 4 and 6 carbon atoms.

9. The serum-separating agent according to claim 6 which contains a structure-forming agent in an amount between 0.04 and 0.6 parts by weight relative to 100 parts by weight of said $\alpha$-olefin-dialkyl maleate copolymer.

10. The serum-separating agent according to claim 6 further containing from 1.0 to 3.0% by weight based on said main component of a wax consisting of an $\alpha$-olefin-dialkyl maleate copolymer whose $\alpha$-olefin component have between 30 and 60 carbon atoms.

**Revendications**

1. Agent de séparation du sérum avec des caractéristiques thixotropes pour séparer un composant sérique par centrifugation de la partie coagulée, caractérisé en ce qu'il contient en tant que composant principal un copolymère d'$\alpha$-oléfine et de maléate de dialcoyle d'une viscosité comprise entre 100 et 800 N·s/m² (à 25°C) et comme composant secondaire un agent de réglage de la viscosité et de la densité, mélangé au composant principal de manière à ajuster la densité de la composition entre 1,035 et 1,055 (à 28°C).

2. Agent de séparation du sérum suivant la revendication 1, caractérisé en ce que le copolymère d'$\alpha$-oléfine et de maléate de dialcoyle répond à la formule générale

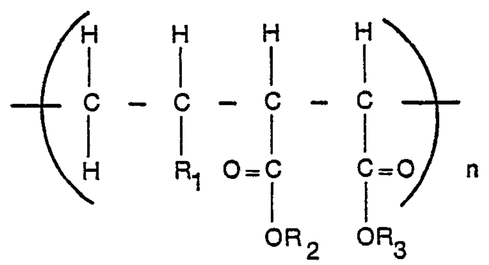

dans laquelle $R_1$ désigne un radical alcoyle ou un mélange de radicaux alcoyle différents en $C_2$ à $C_{58}$, $R_2$ et $R_3$ désignent des radicaux méthyle, éthyle, butyle ou éthyl-2-hexyl et n est un nombre entier.

3. Agent de séparation du sérum suivant la revendication 1 ou la revendication 2, caractérisé en ce que les agents de réglage de la viscosité et de la densité sont choisis parmi les dérivés d'amines aliphatiques d'argile smectique avec 8 à 24 atomes de carbone et des poudres minérales finement divisées.

4. Agent de séparation du sérum suivant la revendication 3, caractérisé en ce que les dérivés d'amines aliphatiques d'argile smectique sont des sels d'ammonium quaternaire d'argile smectique.

5. Agent de séparation du sérum suivant la revendication 3, caractérisé en ce que la poudre minérale finement divisée est de la silice calcinée ou de la silice précipitée.

6. Agent de séparation du sérum suivant la revendication 2, caractérisé en ce que, dans la formule générale, $R_2$ et $R_3$ désignent des radicaux méthyle.

7. Agent de séparation du sérum suivant la revendication 6, caractérisé en ce que, dans la formule générale, $R_1$ représente un mélange de radicaux alcoyle en $C_{10}$ et $C_{12}$.

8. Agent de séparation du sérum suivant la revendication 6, caractérisé en ce que, dans la formule générale, $R_1$ représente un mélange de radicaux alcoyle en $C_4$ et $C_6$.

9. Agent de séparation du sérum suivant la revendication 6, caractérisé en ce qu'il contient, pour 100 parties en poids de copolymère d'$\alpha$-oléfine et de maléate de dialcoyle, entre 0,04 et 0,6 partie d'un additif de structure.

10. Agent de séparation du sérum suivant la revendication 6, caractérisé en ce qu'il contient en outre de 1,0 à 3,0% en poids par rapport au composant principal d'une cire constituée d'un copolymère d'$\alpha$-oléfine et de maléate de dialcoyle avec une composante $\alpha$-oléfine en $C_{30}$ à $C_{60}$.

**Patentansprüche**

1. Mittel zum Trennen von Blutserum mit thixotropen Eigenschaften zum Fraktionieren der Serumkomponente vom koagulierten Anteil durch Zentrifugieren, dadurch gekennzeichnet, daß es als Hauptkomponente ein $\alpha$-Olefin-Dialkylmaleat-Copolymeres mit einer Viskosität von 100 bis 800 N·s/m² (25°C) und im Gemisch damit als Nebenkomponente ein, die Viskosität und das spezifische Gewicht kontrollierendes Agens zum Einstellen des spezifischen Gewichts der Gesamtmischung auf 1,035 bis 1,055 (28°C) enthält.

2. Mittel zum Trennen von Blutserum nach Anspruch 1, worin das $\alpha$-Olefin Dialkylmaleat-Copolymere die allgemeine Formel

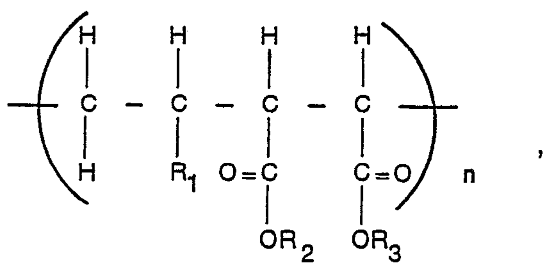

hat, worin $R_1$ eine Alkylgruppe mit 2 bis 58 Kohlenstoff-Atomen ist, die im Copolymer-Molekül entweder die gleiche Gruppe sind oder nach Wunsch eine Kombination verschiedener Gruppen sind, worin $R_2$ und $R_3$ aus Methyl, Ethyl, Butyl- und 2-Ethylhexyl-Gruppen ausgewählt werden und n eine ganze Zahl ist.

3. Mittel zur Abtrennung von Blutserum nach Ansprüchen 1 oder 2, worin die Agentien zur Kontrolle von Viskosität und spezifischem Gewicht aus aliphatischen Aminderivaten von Smektit-Ton mit Kohlenstoff-Atom-Zahlen zwischen 8 und 24 und anorganischen Feinpulvern ausgewählt werden.

4. Mittel zum Trennen von Blutserum nach Anspruch 3, worin die aliphatischen Aminderivate von smektischem Ton quartäre Ammoniumsalze von Smektit-Ton sind.

5. Mittel zum Trennen von Blutserum nach Anspruch 3, worin das anorganische Feinpulver kalziniertes Siliziumdioxid oder präzipitiertes Siliziumdioxid ist.

6. Mittel zum Trennen von Blutserum nach Anspruch 2, worin $R_2$ und $R_3$ der allgemeinen Formel Methylgruppen sind.

7. Mittel zum Trennen von Blutserum nach Anspruch 6, worin $R_1$ in der allgemeinen Formel eine Kombination von Alkylgruppen mit 10 und 12 Kohlenstoff-Atomen ist.

8. Mittel zum Trennen von Blutserum nach Anspruch 6, worin $R_1$ in der allgemeinen Formel eine Kombination von Alkylgruppen mit 4 und 6 Kohlenstoff-Atomen ist.

9. Mittel zum Trennen von Blutserum nach Anspruch 6, enthaltend ein strukturbestimmendes Agens in einer Menge von 0,04 bis 0,6 Gew.-Teilen, bezogen auf 100 Gew.-Teile des $\alpha$-Olefin-Dialkylmaleat-Copolymeren.

10. Mittel zum Trennen von Blutserum nach Anspruch 6, zusätzlich enthaltend 1,0 bis 3,0 Gew.-%, bezogen auf die Hauptkomponente, eines Wachses, bestehend aus einem $\alpha$-Olefin-Dialkylmaleat-Copolymeren, dessen $\alpha$-Olefin-Komponente zwischen 30 und 60 Kohlenstoff-Atomen besitzt.

1

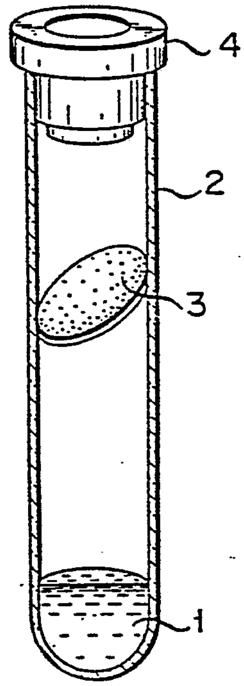


2